(19) Europäisches Patentamt
European Patent Office
Office européen des brevets

(11) Publication number: **0 121 262 B1**

(12) **EUROPEAN PATENT SPECIFICATION**

(45) Date of publication of patent specification: 27.11.91 (51) Int. Cl.⁵: **G01N 21/64**, G01N 15/10, G01N 33/52

(21) Application number: 84103603.1

(22) Date of filing: 31.03.84

(54) Method and apparatus for distinguishing multiple subpopulations of cells in a sample.

(30) Priority: 05.04.83 US 482345

(43) Date of publication of application:
10.10.84 Bulletin 84/41

(45) Publication of the grant of the patent:
27.11.91 Bulletin 91/48

(84) Designated Contracting States:
BE DE FR GB IT NL SE

(56) References cited:
US-A- 3 864 571
US-A- 3 916 197
US-A- 3 916 205
US-A- 4 284 412

JOURNAL OF IMMUNOLOGICAL METHODS,
vol. 50, 1982, pages 193-204, Elsevier
Biomedical Press; J.A. TITUS et al.: "Texas
Red, a hydrophilic, red-emitting fluorophore
for use with fluorescein in dual parameter
flow microfluorometric and fluorescence mi-
croscopic studies"

(73) Proprietor: Becton, Dickinson and Company
Mack Centre Drive P.O. Box 2224
Paramus New Jersey 07652-1149(US)

(72) Inventor: Loken, Michael R.
29 San Juan Court
Los Altos California 94022(US)
Inventor: Parks, David R.
68 Albion Street
San Francisco California 94103(US)
Inventor: Hardy, Richard R.
2251 Harvard Street
Palo Alto California 94306(US)

(74) Representative: von Kreisler, Alek,
Dipl.-Chem. et al
Deichmannhaus am Hauptbahnhof
W-5000 Köln 1(DE)

THE JOURNAL OF CELL BIOLOGY, vol. 93, June 1982, pages 981-986, The Rockefeller University Press; V.T. OI et al.: "Fluorescent phycobiliprotein conjugates for analyses of cells and molecules"

THE JOURNAL OF HISTOCHEMISTRY AND CYTOCHEMISTRY, vol. 25, no. 7, 1977, pages 836-844, The Histochemical Society, Inc., New York, US; H.M. SHAPIRO et al.: "Cytomat-R: A computer-controlled multiple laser source multiparameter flow cytophotometer system"

THE JOURNAL OF HISTOCHEMISTRY AND CYTOCHEMISTRY, vol. 25, No. 7, 1977, pages 899-907, The Histochemical Society, Inc., New York, US

## Description

### BACKGROUND OF THE INVENTION

1. Field of the Invention The present invention relates to a method and apparatus for distinguishing multiple subpopulations of particles in a sample, and more particularly, concerns a method and apparatus for distinguishing and quantifying a greater number of different immunofluorescent stains in a single cell population than the number of light sources available to provide excitation energy to the fluorescent stains.

2. Description of the Prior Art Recent cytometric analysis of cells, particles and the like has emphasized the utilization of cell surface markers or immunofluorescent stains to study the distribution of antigens on various subpopulations of cells. Cells to be studied are labeled with immunofluorescent stains bearing different fluorochromes in order to correlate the antigenic determinants on these cells or particles. As used herein, the term "immunofluorescent stains" refers generally to a chromophore physically attached to another molecule which adds specificity to the attachment of the chromophore to a target. An example of this procedure is the coupling of a fluorochrome to antibodies or to hormones. This type of fluorescent staining, accordingly, is quite different from the known staining in which the specificity of the reaction is imparted by the chromophore itself. An example of this staining is the reactivity of certain dye molecules with DNA in which internal elements of the cells are stained with different dyes in order to assess characteristics of those cells.

One of the difficulties encountered in using immunofluorescent stains in a flow cytometry device relates to the ability to detect multiple stains on cells in a sample. Presently known and available flow-through cytometers useful for detecting particles, cells and the like, commonly include two channels for the detection of two independent stains on cells in a mixture. For example, devices are known which include two fluorescence channels which can detect cells specifically labeled with two cell surface markers, such as immunofluorescent stains, associated with the respective fluorescence channels. In these known devices, a complete fluorescence channel including the electrical circuitry and fluorescense detector has been required for each category of fluorescent-reactive cells to be detected in the mixture of cells in the sample being analyzed. Therefore, in order to detect multiple subpopulations of cells in a sample using flow-through cytometry, an equivalent number of fluorescence channels is employed using the known, conventional devices. If only one light source, such as a laser is used, a dual-pass protocol can be used to sequentially assess two antigenic determinants. However, it has been known in the art that a single light source can be used as an excitation source for two fluorescent dyes or stains. For example, cells labeled with both fluoresce in and rhodamine have been analyzed so that the emission from either chromophore was detected independently even though the emission spectra of the dyes overlap. A simple wavelength of excitation was used for the excitation of the two dyes. This achievement was described by Loken, M.R., Parks, D.R. and Herzenberg, L.A., "Two-Color Immunofluorescence Using a Fluorescence-activated Cell Sorter," The Journal of Histochemistry and Cytochemistry, Vol. 25, No. 7, pp. 899-907, 1977. Recent developments in immunofluorescent stains have improved the single laser excitation of two stains. The use of phycoerythrin and fluorescein as dye pairs in immunofluorescence has been described by Oi et al., in "Fluorescent Phycobiliprotein Conjugates for Analyses of Cells and Molecules," The Journal of Cell Biology, Vol. 93, pp. 981-986, June 1982.

However, presently available flow cytometry devices generally employ two lasers in order to excite two distinguishable fluorescent markers. Consistent with this limitation is the fact that a separate light source, such as a laser, is generally the mechanism relied upon to excite each different type of immunofluorescent stain which has been tagged onto a cell or particle to be studied. Thus, in order to analyze or quantify two different immunofluorescent stains in a single sample, two lasers providing excitation energy at significantly separated wavelengths are normally employed. Apparatuses utilizing two lasers for analyzing an equivalent number of immunofluorescent stains are described, for example, in U.S. Pat. Nos. 3,826,364 and 4,284,412. Use of two lasers in a flow system, although not related to immunofluorescence, was also described in Stohr, M., "Double Beam Application in Flow Techniques and Recent Results," in Proceedings of the Second International Symposium on Pulse-Cytophotometry, Golde, W., Schumann, J., Bucker, T., eds., European Press, Ghent, 1976, pp. 39-45.

Specifically, if two lasers are to be utilized in a flow cytometry device, stains having sufficiently separated excitation and emission spectra, as well as lasers having sufficiently separated primary emissions, should be chosen, if available. Immunofluorescent stains that may be used to bind to cells and which are well-suited for two-color two laser flow cytometric analyses are fluorescein and Texas Red. The use of Texas Red as an immunofluorescent stain has been described by Titus, J.A., in "Texas Red, a Hydrophilic, Red-Emitting Fluorophore for Use with Fluorescein in Dual Parameter Flow Microfluorometric and Fluorescence Microscopic Studies," Journal of Immunological Methods, 50 (1982), 193-204.

Improvements in the availability of lasers to provide more optimum matching of the excitation energy lines to the excitation spectrum of the second fluorophore have been reported. Use of a dye laser as a second laser in a dual laser flow system provides emissions to excite a second fluorophore. Such a system has been described by Arndt-Jovin, D.J., et al., in "A Dual Laser Flow Sorter Utilizing a CW Pumped Dye Laser, Cytometry, Vol. 1, No. 2, pp. 127-131, 1980.

However, the need to assess three, four and even more antigenic determinants in a single pass through the flow cytometer has become a sought-after goal as more research is being performed on heterogeneous cell populations.

A three-laser flow cytometer has been described wherein three fluorescent dyes (of the DNA-staining type), having different excitation spectra, have been detected on cells. Steinkamp, J.A., et al., "Three-Color Fluorescence Measurements on Single Cells Excited at Three Laser Wavelengths," Cytometry, Vol. 2, No. 4, pp. 226-231, 1982. Another description of using multiple light sources is found in Shapiro, H.M., et al., "Cytomat-R: A Computer-Controlled Multiple Laser Source Multi-parameter Flow Cytophotometer System," The Journal of Histochemistry and Cytochemistry, Vol. 25, No. 7, pp. 836-844, 1977. However, the need, as alluded to above, is to minimize the number of light sources which are required to provide excitation energy, while yet being able to analyze multiple subpopulations of cells. To this end, each light source would desirably excite more than one fluorescent marker. However, when two light sources are employed, problems arise relating to the spectral spacing of the immunofluorescent stains which have been available. In order to avoid the expense, complications and complexities of adding new light sources for each antigenic determinant under study, it would be most advantageous to use light sources which excite multiple immunofluorescent stains. This advantageous feature was demonstrated to be practical by Loken et al.

There are many instances when it is desirable to be able to detect multiple subpopulations of cells from a sample mixture with a single pass of the cells through the region where analysis occurs. Such analysis desirably includes the assessment of subpopulations in region where there is an overlap of cell parameters under study. For instance, in performing certain tests on blood, it may be desirable to detect or quantify the proportions of T-cells, B-cells and monocytes in a single pass of cells through a flow-through cytometer. Similarly, the detection and quantification of other multiple subclasses of leukocytes may also be desired. Clearly, this has created formidable problems. While it is desirable to be able to detect, and also quantify, multiple subpopulations of cells from a sample mixture, it is even more desirable to minimize the number of light sources, such as lasers, and the associated circuitry. With this in mind, the present invention is directed to solving the aforementioned problems while satisfying the desired need for the determination of multiple labeled subpopulations of cells from a sample mixture, with little compromising of any of the fluorescence signals.

Above-mentioned Journal of Cell Biology, Vol. 93, June 1982, pp. 981-986, Elsevier Biomedical Press discloses a method for distinguishing and determining of multiple subpopulations of biological cells in a sample by immunofluorescent methods, the method comprising the following step:

(a) labelling biological particles with a plurality of different surface marking agents responsive to light stimulation;

(b) passing such labelled biological particles through a plurality of spacially separated areas, and

(c) detecting the characteristics of different biological particles labelled with different surface marking agents simultaneously by optical (including immunofluorescent) methods and thereby distinguishing the particles from each other.

As explained in detail in the passage bridging the left and right column on page 983, the fluorescence-activated cell analysis was performed using a modified Becton, Dickinson & Co. fluorescence activated cell sorter which requires an electronic compensation as dealt with in detail in the fifth line in the right hand column referring to a publication by Locan et al in the Journal of Histochemistry and Cytochemistry, Vol. 25, No. 7, pp. 899-907, 1977. In other words: The Journal of Cell Biology requires a combination of optical and electronical means for the separation and detection of the fluorescence of the two light beams.

SUMMARY OF THE INVENTION

A method for distinguishing multiple subpopulations of particles in a sample comprises selectively labeling particles in a sample with plurality of different surface marking agents. Each marking agent has distinguishing, quantifiable marking characteristics. The labeled particles are passed, substantially one at a time, successively through a plurality of areas of detection to detect the characteristics of one or more different marking agents on particles in each area of detection. A greater number of marking agents are detected than the number of detection areas through which the particles pass. This method includes distinguishing differences among the particles relative to the detected, different quantifiable characteristics of said marking agents.

In a preferred embodiment of this aspect of the present invention, the method is defined in independent claim 1.

Another aspect of the present invention is an apparatus as defined in claim 11.

Further embodiments of the method and the apparatus can be found in the dependent claims.

In accordance with the principles of the present invention, a number of advantages and objectives are attained. Primarily, the present invention permits the detection and quantification of multiple subpopulations of particles or cells in a greater quantity than the number of light sources, such as lasers, employed. Thus, a greater number of cell subpopulations can be determined in a single pass through a flow cytometer because the present invention utilizes a feature wherein a single light source, such as a laser, can excite more than one immunofluorescent stain or the like. By selecting fluorescent surface labels which, though sufficiently separated in excitation wavelength to provide distinguishing characteristics, are close enough in spectral range to be excited by a single light source, dual color immunofluorescence results comparable to the two laser excitation mechanism can be achieved. When this approach is employed, it is feasible to simultaneously quantify at least three different immunofluorescent stains in a single cell population by using only two lasers, and it is possible to extend this approach to detect four or more cellular immunofluorescent stains.

## BRIEF DESCRIPTION OF THE DRAWINGS

Fig. 1 is a schematic illustration of a preferred embodiment of the optical elements and light paths of a flow cytometry device particularly useful for determining fluorescence related to multiple subpopulations of particles and light scatter parameters of particles flowing in a flow path;

Fig. 2 is a graphic representation of the detection and quantification of three subpopulations of leukocytes in a sample determined by a single pass through a flow cytometer incorporating two lasers therein in accordance with the principles of the present invention; and

Figs. 3-5 are graphic representations of correlation plots of fluorescence parameters of the cells which have been detected.

## DETAILED DESCRIPTION

While this invention is satisfied by embodiments in many different forms, there is shown in the drawings and will herein be described in detail a preferred embodiment of the invention, with the understanding that the present disclosure is to be considered as exemplary of the principles of the invention and is not intended to limit the invention to the embodiment illustrated. The scope of the invention will be measured by the appended claims and their equivalents.

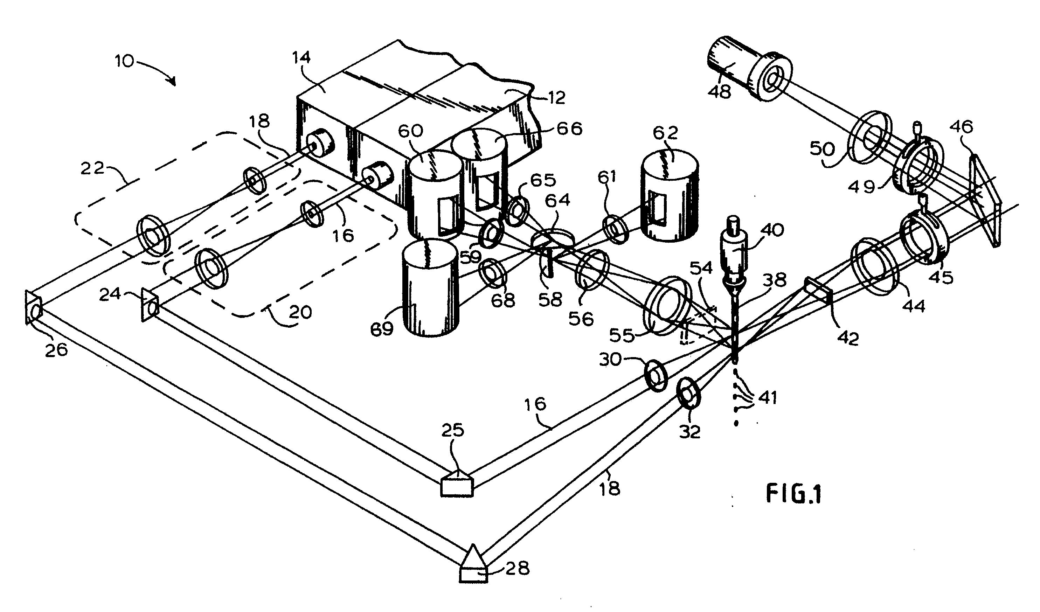

Adverting to the drawings, and Fig. 1 in particular, the optical and particle flow elements of a flow cytometry device 10 are illustrated. The optical and flow elements of Fig. 1 represent the major components of a flow cytometry instrument for flowing particles in a liquid stream, substantially one at a time, in order to analyze those particles for specific characteristics thereof. For example, the elements of the device of Fig. 1 may be included in a FACS fluorescence-activated sorter, manufactured and sold by the FACS Systems Division of Becton, Dickinson and Company, Sunnyvale, California. The FACS cell sorter analyzes and separates cell populations on the basis of light scatter and fluorescence in a wide variety of research laboratory applications. In addition to the optical and flow elements to be described in more particular detail herein, and which may be embodied in an instrument such as the FACS cell sorter, other details of a cell sorting apparatus useful in conjunction with the present invention are described in U.S. Pat. No. 3,826,364. It is understood that the present invention is useful in many different types of flow cytometry devices, whether measuring light scatter, particle volume, fluorescence or any other optical parameters for the identification or quantification of subpopulations of particles in a sample. The optical elements, in particular, of the present invention represent the essence of the improvement in flow cytometry devices such as described in the aforementioned patent.

As illustrated in Fig. 1, light energy is provided for the present flow cytometry device by two lasers 12 and 14. In this embodiment being described, two sources of light are provided in flow cytometry device 10 so that it is possible to detect and quantify a plurality of different types of particles having different fluorescence characteristics. It is understood, however, that the inclusion of two lasers in the embodiment illustrated in Fig. 1 is merely preferable and serves as an exemplary embodiment of employing more than one light energy source and analysis elements in the type of invention being described.

In the present invention, lasers 12 and 14 are selected to produce primary emissions of coherent light at specific wavelengths separated from each other in the spectral range. For example, laser 12 is preferably selected to operate in the blue/green spectral region whereby fluorochromes, which fluoresce when illuminated by optical stimulation of certain wavelengths, attached to particles passing through the light generated by laser 12 will become excited. One such laser useful for the present invention is an argon ion laser having a primary emission at 488 nanometers (nm). Laser 14 is preferably selected to operated at a different, separated wavelength from laser 12. Particles having fluorochromes thereon which fluoresce when illuminated by optical stimulation of the wavelength of laser 14 will become excited when these particles pass through the light generated by laser 14. The operation of laser 14 may cover the yellow/red region of the visible spectrum so as to be substantially separated in wavelength from the spectral region of laser 12. One such laser which satisfies this requirement is a rhodamine 6-G dye laser which has a primary emission at 600 nm. Another laser which has a primary emission (633 nm) sufficiently separated from the argon ion laser and which could serve as the second laser in the present invention is a heliumneon laser. Other lasers, if available under the criteria described herein, may also be selected. It is desirable that the difference in wavelengths of the two lasers be substantial enough to be outside of the spectral range of excitation and emission of at least two immunofluorescent stains to be excited with each laser. It has been found that a difference in wavelengths between the two lasers of 75 to 100 nm, or greater, permits the desirable result just described. Of course, the selection of the immunofluorescent stains or other quantifiable surface marking agents must be compatible with the lasers which are used in the present invention so that two or more immunofluorescent stains may be excited by the light energy provided by each laser.

Emerging from lasers 12 and 14, each beam 16 and 18, respectively, may pass through beam expanders schematically indicated by numerals 20 and 22 which enlarge each beam while retaining the parallel character thereof. As each beam emerges from the beam expander, which is preferable but not necessary for operation of the present invention, it normally must have its direction changed due to space requirements in the flow cytometry device. To this end, beam 16 is reflected through prisms 24 and 25, while beam 18 is reflected through prisms 26 and 28. All of these prisms may be made adjustable to properly align the beams during operation.

After beams 16 and 18 pass through the prisms, they are directed toward lenses 30 and 32 for focusing the beams onto the stream of particles.

While lenses 30 and 32, if employed, are selected in accordance with the type of flow cytometry device being employed, these lenses may be selected in accordance with the description thereof in patent application, serial number 361,672, filed in the U.S. Patent and Trademark Office on March 25, 1982, and having a common assignee as the present application.

Once the laser beams pass through lenses 30 and 32, they are directed onto particle stream 38. A nozzle 40, incorporated within the flow cytometry device of the present invention facilitates the flowing of particles 41 within fluid stream 38. The utilization of a nozzle of this type is well-known and is described, for example, in U.S. Pat. No. 3,826,364. In the present device being described, the two laser beam-particle stream intersections are spaced approximately 250 micrometers apart. As seen more clearly in Fig. 1, laser beam 16 lies on the optical axis of a light-scatter channel and is used for scatter detection of particles. It is understood, however, that the light scatter features herein described are merely included to round out the features of a typical flow cytometry device which may rely upon light scatter to obtain information from particles passing through the light beams.

Thus, light beam 16 is the first light beam encountered by a particle flowing in stream 38 emerging from nozzle 40. Thereafter, beam 16 strikes the light-scatter obscuration bar 42 on the optical axis of the light-scatter channel. Scattered light, collected by the lens 44, passes through a first iris 45 which determines the maximum angle of scattered light collected. Following first iris 45 is a beam splitting mirror 46 which reflects a percentage of the incident light toward scatter detector 48, and transmits the remaining percentage of the incident light onto a light absorber (not shown). A second iris 49 functions as a field stop to restrict the source of scattered light to the point of intersection of laser beam 16 and stream 38. After passing through filter 50, the scattered light is detected in detector 48. This detector functions electrically to assess the size of the particles flowing in the fluid stream according to well-known techniques.

In the embodiment of the present invention illustrated in Fig. 1, laser beam 18 is also directed at flowing stream 38, but is vertically displaced from laser beam 16 along the vertical axis of the stream. Light from beam 18 scattered by a particle is picked up by the scatter-channel optics, but preferably blocked from detector 48 by the dielectric filter placed in the scatter channel.

With respect to the fluorescence channels, illumination provided by the different wavelength operation of the lasers is available for sequential excitation of fluorochromes having predetermined, substantially separated emission spectra. At least one, but preferably two or more immunofluorescent stains having different emission spectra are excited by the excitation energy provided by the light energy of each laser. As seen in Fig. 1, the two independent laser beams intersect stream 38 at points vertically spaced so that a particle crosses laser beam 16 first and then laser beam 18. Accordingly, two pairs of optical signals may be generated by the particles passing through the light beams. These pairs of signals are preferably spaced in time by the time required for the particle to travel from the first beam intersection point to the second beam intersection point. This time spacing permits the pairs of signals to be separately analyzed giving signals proportional to the fluorescence emissions of the particles when excited at the two different excitation wavelengths. Fluorescence signals emitted from the particles are directed around obscuration bar 54 which blocks refracted light from the separated beams. All fluorescence signals are focused by lens 55 preferably through a first filter 56 which permits light only at specific wavelengths to pass therethrough.

Fluorescence emitted by particles stimulated by light beam 16, after passing through filter 56, then encounters a dichroic mirror 58. The purpose of dichroic mirror 58 is to separate two different colors traveling along the fluorescence light path so that they can be analyzed separately. For example, dichroic mirror 58 would be selected to separate, for example, the different color wavelengths of particles excited by light beam 16 produced only by laser 12. For instance, wavelengths in the green region would be transmitted through dichroic mirror 56 and then through a barrier filter 59 which is designed to transmit wavelengths of only one color region, in this instance green. Green light then enters a fluorescence detector 60.

Light encountering dichroic mirror in the orange color region would be reflected by the dichroic mirror through a barrier filter 61 which transmits wavelengths of only one color region, in this case, orange. A fluorescence detector 62 then receives this orange light.

Light beam 18 also provides excitation energy at a single wavelength sufficient to excite up to two fluorochromes different from those excited by light beam 16. After the fluorescence emitted by the particles excited by light beam 18 passes through lens 55 and first filter 56, this light encounters another dichroic mirror 64. Similar to the description of dichroic mirror 58, the second dichroic mirror 64 is selected to separate the wavelengths of two different regions of the color spectrum. For example, both red and far red signals may be generated as a result of the single excitation source provided by light beam 18 directed from laser 14. Wavelengths in the red region would be transmitted through dichroic mirror 64 and then through a barrier filter 65 which is designed to transmit wavelengths of only the red region. This light is then directed to a fluorescence detector 66. Wavelengths in the far red region would be reflected by dichroic mirror through a barrier filter 68, whereupon this light enters fluorescence detector 69. Accordingly, it can be seen that light from two lasers, each of which can excite two or more different fluorochromes at the respective wavelengths of operation of each, allows the detection and quantification of multiple subpopulations of particles in a sample during a single pass of that sample in a flow cytometry device such as described.

Fluorescence detectors 60, 62, 66 and 69 are provided to preferably receive the four separated green, orange, red and far red light paths, respectively. These fluorescence detectors may be low-noise photomultiplier tubes or the like which convert optical signals into electrical signals. Although not shown in Fig. 1, these electrical signals are then fed electrically to be processed by the electronics of the flow cytometry device for analysis or other purposes. Various displays, information presentation, accumulation or recordation may be provided in the flow cytometry device. Similarly, the particles having specific characteristics may be separated and sorted in accordance with the technique taught in U.S. Pat. No. 3,826,364.

Operation of the present invention shall now be described in conjunction with the following example for illustrative purposes only. This example exemplifies, but does not limit the scope of, the technique for detecting, distinguishing and/or quantifying multiple subpopulations of particles in a sample.

In this example, mononuclear leukocytes obtained from a human source were prepared in known fashion and reacted with three monoclonal antibodies with the designations anti-leu 2, anti-leu 7 and anti-leu 11 having three different immunofluorescent stains labeled thereon. These three immunofluorescent stains each have a predetermined fluorescence response to an optical stimulation. In particular, anti-leu 2 was conjugated with phycoerythrin (PE), to form an immunofluorescent stain which, when stimulated, emits fluorescence at a wavelength of about 575 nm, at or near the orange region of the color spectrum. Anti-leu 7, coupled with biotin and reacted in indirect labeling with avidin-conjugated with Texas Red (trademark of Molecular Probes, Plano, Texas) which, when stimulated, emits fluorescence at a wavelength of about 620 nm, placing it at or near the red region of the color spectrum. Anti-leu 11 was conjugated with a fluorochrome known as fluorescein (FITC) to form an immunofluorescent stain which, when excited, emits fluorescence at a wavelength of approximately 530 nm, placing it in the green region of the color spectrum.

These mononuclear leukocytes labeled sequentially with anti-leu 2 (PE), anti-leu 11 (FITC), and anti-leu 7 (biotin and avidin Texas Red) were placed in a sample liquid medium and the sample was passed through a dual laser FACS fluorescence-activated cell sorting flow cytometry device (Becton Dickinson FACS Systems, Sunnyvale, California). Two lasers were utilized in an arrangement such as illustrated in Fig. 1 herein. One of the lasers was an argon ion laser having a primary emission at 488 nm; the other laser utilized was a rhodamine 6-G dye laser having a primary emission at 600 nm. Utilizing the arrangement such as illustrated in Fig 1, the labeled leukocyte cells were passed, substantially one at a time, succesively through two areas of optical stimulation, one such area stimulated by light from the argon ion laser, the other area stimulated by light from the rhodamine 6-G dye laser. FITC and PE, labeled respectively on anti-leu 11 anti-leu 2, were both excited by light from the argon ion laser, and fluorescence emitted by these stains was detected by the fluorescence detectors 60 and 62, respectively. Light from the rhodamine 6-G dye laser stimulated the Texas Red carried on anti-leu 7. The fluorescence emitted thereby was detected by fluorescence detector 66.

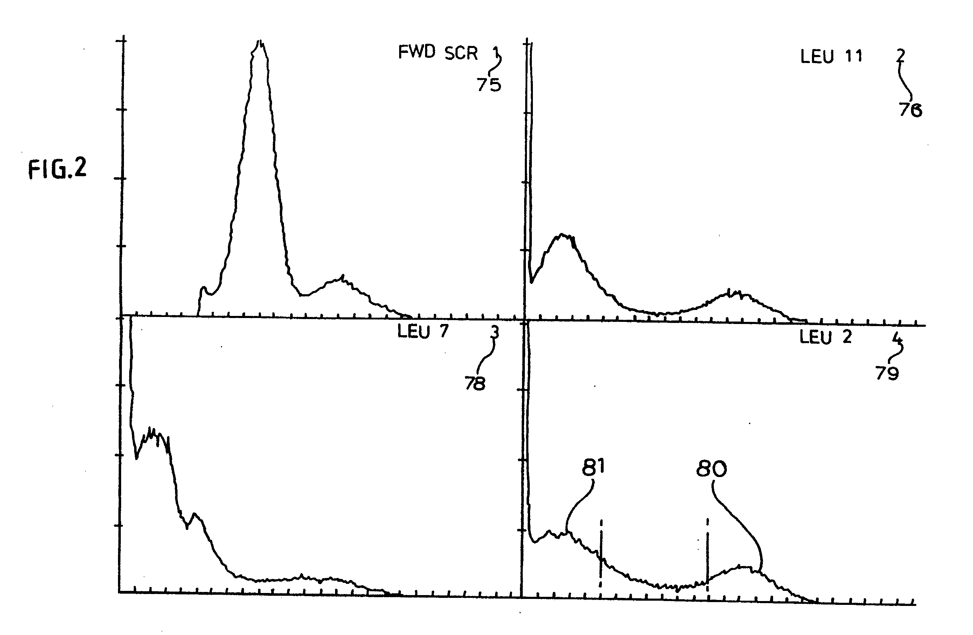

The optical signals provided to fluorescence detectors 60, 62 and 66, as well as the scatter signal of the particles passing through the light beams were provided to the analysis controls of the FACS equipment and were stored as a four-parameter list so that the results could be displayed and interpreted. As illustrated in Fig. 2, four histograms are displayed of ungated data which show the results of this experiment to distinguish the multiple subclasses of leukocytes labeled with three different antibodies in one pass through the flow cytometry device. The first channel 75 displayed the forward scatter results of the particles passing through the 488 nm light beam and can be used to distinguish lymphocytes from monocytes based on their size. In the second channel 76, the histogram of the anti-leu 11 staining was displayed from the fluorescence signals generated thereby. Similarly, the third channel 78 displayed the histogram of the anti-leu 7 labeling of cells. In the fourth channel 79, the histogram of the anti-leu 2 labeling of cells was displayed as a function of their fluorescence characteristics.

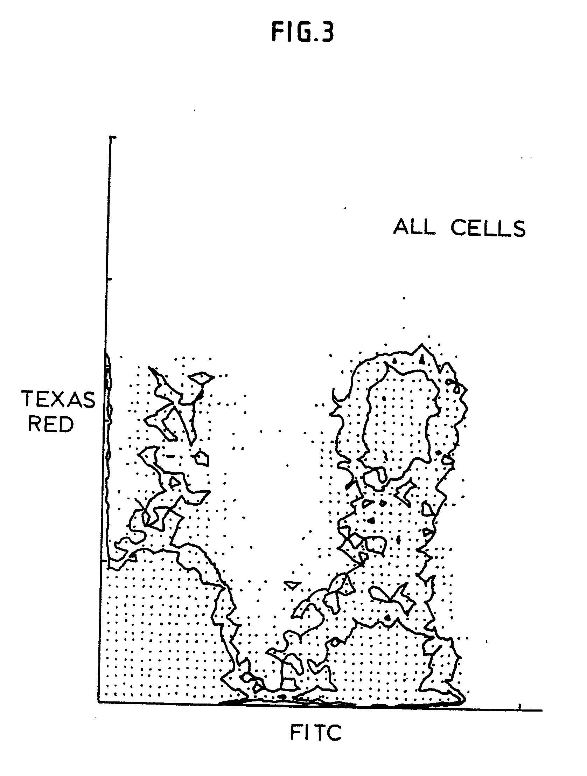

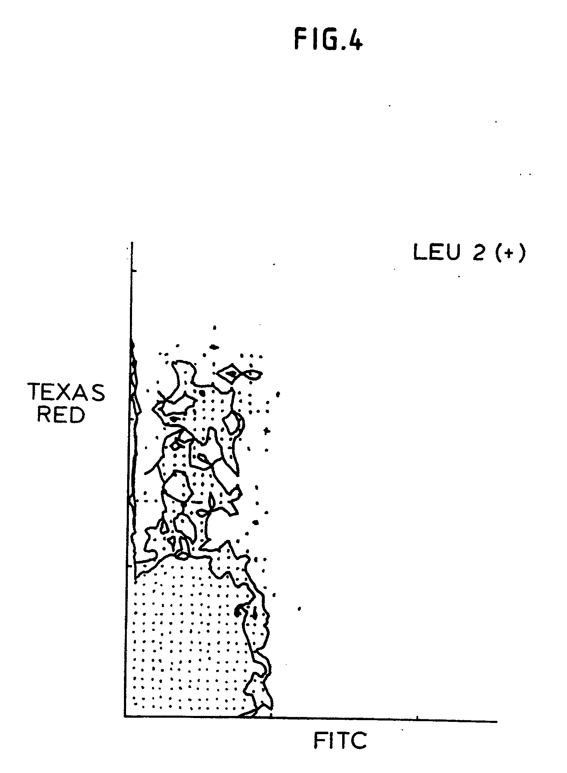

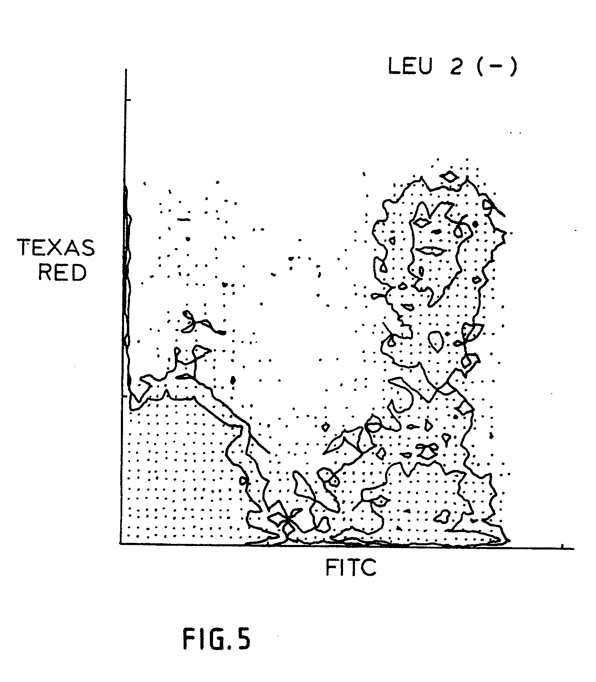

A correlation plot of the two cell surface markers leu 7 and leu 11 is illustrated in Fig. 3. With respect to this figure, cells with light scatter signals identifiable as lymphocytes (from Fig. 2) were displayed. Upon reanalysis of this same data based on the additional parameter of leu 2, Figs. 4 and 5 were generated. The correlation of anti-leu 7 and anti-leu 11 staining of those cells which expressed the leu 2 antigen (gated area 80 of Fig. 2) is shown in Figure 4. Similar correlation plot of the cells which did not bind anti-leu 2 antibody (gated area 81 of Fig. 2) is illustrated in Fig. 5.

Accordingly, the reactivity of three different antibodies on leukocyte cells were detected, distinguished and quantified based on the detected, different characteristics of the immunofluorescent stains labeled on each cell as it passed through the instrument. While three different antibodies were detected in association with three different fluorochromatic agents, only two coherent light sources were relied upon in generating these results.

In order to quantify four different immunoflourescent stains in conjunction with the present invention, a second fluorochrome, distinguishable from Texas Red, but excitable by the rhodamine 6-G dye laser, or other appropriate laser, could be employed. Specifically, if a fourth subpopulation of leukocytes were labeled with another immunofluorescent stain, having as a conjugate the fluorochrome known as allo phycocyanin (which emits fluorescence at a wavelength of approximately 680 nm when excited placing it in the far red region of the color spectrum), four different immunofluorescent stains could be quantified by the present invention. Instead of Texas red, the fluorochrome known as R-phycocyanin may be used with results analagous to those obtained using Texas Red. Still other fluorochromes may be employed to expand the analysis of multiple subpopulations of particles or cells.

While the above example for distinguishing three subpopulations of leukocytes, along with a modification thereof for assessing four subpopulations of cells while only using two lasers, specifically identifies the four immunofluorescent stains utilized, it is understood that the present invention is not limited to such a combination as described.

Moreover, if the flow cytometry device has the appropriate resolution and data accumulation capabilities, the use of two lasers and three distinguishable immunofluorescent stains could permit the identification of up to eight different subsets of particles or cells. This same type of geometric progression will characterize the utilization of two lasers with four or more distinguishable immunofluorescent stains, so that many subsets can be identified in the analysis of particles or cells.

Thus, the present invention provides a method and apparatus for detecting and distinguishing multiple subpopulations of particles in a sample. Advantageously, more subpopulations of particles in a sample may be distinguished in one pass of particles through the flow cytometry device than the number of fluorescence excitation sources employed in this invention. This feature clearly increases the efficiency of using flow cytometry techniques to detect, quantify and sort multiple subpopulations of cells or the like particles.

## Claims

1. A method for the simultaneous correlated detection of different biological particle surface antigens in a sample, comprising
   - selectively labelling biological particle surface antigens of biological particles in a sample with a plurality of different surface marking agents responsive to light stimulation, each marking agent having spectrally distinguishable, quantifiable marking characteristics measurable by its light output,
   - passing said labeled biological particle surface antigens successively through a plurality of spacially separate areas, each area including a beam of light at a different wave length, the light in one area being at a wavelength which causes the stimulation of multiple surface marking agents on biological particle surface antigens passing through said one area, but which wavelength is sufficiently spectrally separate from the wavelength of light in an adjacent area so as to be substantially outside of the spectral range of stimulation and light output of the surface marking agents stimulated in an adjacent area;
   - detecting the characteristics of a greater number of surface marking agents than the number of said areas through which particles pass and said detection being performed simultaneously with respect to a plurality of characteristics associated with different surface marking agents in anyone of said areas; and
   - distinguishing different surface antigens among said biological particle surface antigens relative to the detected, different and separately quantifiable characteristics of said surface marking agents,
   said method being characterized by
       (a) said labeled biological particles passing at least two spacially separate areas substantially one at a time, and
       (b) said detection including separating the surface marking agent's output into distinguishable wavelengths only by first optical means (58,64) and second optical means (59,61,65,68).

2. The method of claim 1 wherein said biological particle surface antigens are cell surface antigens and wherein said method comprises
   - selectively labeling cell surface antigens in a sample with at least three different immunofluorescent stains, each stain having a different fluorochrome with predetermined, substantially separate emission spectra;
   - passing said labeled cell surface antigens, substantially one at a time, through a first area of focused optical stimulation to provide excitation energy to excite one or more of said different stains;
   - sequentially passing said labeled cell surface antigens, substantially one at a time, through a second area of focused optical stimulation to provide excitation energy to excite, in said second area, one or more of said different stains so that at least three different stains are excited by passing through said two areas of optical stimulation, the focused optical stimulation in each of said areas being caused by providing light at a different wavelength to each of said areas, the light which is provided to one of said areas of light stimulation being at a wavelength which causes the excitation of multiple stains on cell surface antigens passing through said one area of light, but which wavelength is sufficiently spectrally separate from the wavelength provided to said other area of light stimulation so as to be substantially outside of the spectral range of excitation and emission of the stains excited in said other area of stimulation;
   - detecting the fluorescence emitted by each differently excited stain by said first optical means (58,64) and by said second optical means (59,61,65,68) separating stain emissions and said detection being performed simultaneously when a plurality of fluorescence signals is emitted from different stains excited in any one of said areas of stimulation; and
   - distinguishing said cell surface antigens relative to the detected fluorescence characteristics thereof.

3. The method of Claims 1 or 2 wherein the first optical means (58,64) are dichroic mirrors and the second optical means (59,61,65, 68) are filters.

4. The method of Claim 2 wherein the three different fluorochromes of the stains are fluorescein, phycoerythrin and Texas red or fluorescein, phycoerythrin and allo phycocyanin.

5. The method of Claim 2 wherein the three different fluorochromes of the stains are fluorescein, phycoerythrin and R-phycocyanin.

6. The method of Claims 1 and 2 wherein said cells are labeled with four different stains and there are two areas of focused optical stimulation.

7. The method of Claim 6 wherein the four different fluorochromes of the stains are fluorescein, phycoerythrin, Texas red and allo phycocyanin.

8. The method of Claim 6 wherein the four different fluorochromes of the stains are fluorescein, phycoerythrin, R-phycocyanin and allo phycocyanin.

9. The method of Claims 1 to 8 wherein the focused optical stimulation in each of said areas is caused by providing coherent light at a given wavelength to each of said areas.

10. The method of Claim 9 wherein the coherent light provided to an area of stimulation is at a wavelength sufficiently separated from the wavelength of an adjacent spectral region of stimulation to be substantially outside of the spectral range of excitation and emission of the stains in an adjacent region.

11. An apparatus for the simultaneous correlated detection of different cell surface antigens which have been selectively labeled with different immunofluorescent stains each having predetermined, substantially separate emission spectra comprising:
    - means (40) for moving said labeled cell surface antigens in a flow path (38);
    - first means (12) including a first source of light (12) for exciting one or more immunofluorescent stains in a first area of optical stimulation along said flow path (38);
    - second means (14) including a second source of light (14) for exciting a plurality of said different immunofluorescent stains in a second area of optical stimulation along said flow path (38), said first and second sources of light selected to provide light at different wavelengths relative to one another, each of which are selected to cause the excitation of multiple stains on cell surface antigens passing through the respective areas of stimulation, but which are sufficiently spectrally separate from each other to be substantially outside of the spectral range of excitation and emission of the excited stains in each of the respective areas of stimulation; and
    - means for distinguishing cell surface antigens relative to the detected fluorescence characteristics thereof,
said apparatus being characterized in that said means (40) is arranged for moving said labeled cell surface antigens in a flowpath (38) substantially one at a time and said apparatus further comprises means (60,62,66,69) for detecting the fluroescence emitted by each differently excited immunofluorescent stain associated with the cell surface antigens including means for separating a plurality of spectrally different fluorescence signals of distinguishable wavelengths emitted from different stains excited by any one of said excitation means, said means for separating only comprising first optical means (58,64) and second optical means (59,61, 65,68).

12. The apparatus of Claim 11 wherein the first optical means (58, 64) are dichroic mirrors and the second optical means (59,61,65,68) are filters.

13. The apparatus of Claim 11 wherein the first means (12) and the second means (14) for exciting each includes a source for providing coherent light to said respective areas of stimulation.

14. The apparatus of Claim 13 wherein the sources (12,14) of coherent light are selected to provide light at wavelengths sufficiently separated from each other to be substantially outside of the spectral range of excitation and emission of the stains in each of the respective areas of stimulation.

15. The apparatus of Claim 13 wherein the first source (12) of coherent light is an argon ion laser and the second source (14) of coherent light is a rhodamine 6-G dye laser or a heliumneon laser.

16. The apparatus of claims 13 and 14 wherein the means for detecting includes up to two photodetector devices associated with each of the first and second means for exciting, each such photodetector device capable of detecting light in a specifically defined color region.

17. The apparatus of Claim 13 wherein the means for distinguishing includes display means for indicating the detected subpopulations of cells.

18. The apparatus of Claim 13 which further includes means to determine the approximate number of cells detected in each subpopulation.

## Revendications

1. Procédé de détection en corrélation simultanée d'antigènes de surface de particules biologiques différents dans un échantillon, dans lequel

   - on marque de façon sélective des antigènes de surface de particules biologiques dans un échantillon comportant plusieurs agents de marquage de surface différents réagissant à une stimulation lumineuse, chaque agent de marquage ayant des caractéristiques de marquage quantifiables pouvant être distinguées spectralement, mesurables par son émission de lumière,
   - on fait passer lesdits antigènes de surface de particules biologiques marqués successivement à travers plusieurs zones spatialement séparées, chaque zone incluant un faisceau de lumière à une longueur d'onde différente, la lumière dans une zone étant à une longueur d'onde qui provoque la stimulation d'agents de marquage de surface multiples sur les antigènes de surface des particules biologiques qui passent à travers ladite zone, mais cette longueur d'onde étant suffisamment séparée spectralement de la longueur d'onde de la lumière dans une zone adjacente pour être sensiblement à l'extérieur de l'intervalle spectral de stimulation et d'émission de lumière des agents de marquage de surface stimulés dans une zone adjacente;
   - on détecte les caractéristiques d'un plus grand nombre d'agents de marquage de surface que le nombre desdites zones à travers lesquelles des particules passent, et ladite détection étant effectuée simultanément quant à plusieurs caractéristiques associées à différents agents de marquage de surface dans l'une quelconque desdites zones; et
   - on distingue différents antigènes de surface parmi lesdits antigènes de surface de particules biologiques quant aux caractéristiques détectées, différentes et séparément quantifiables desdits agents de marquage de surface,

   ledit procédé étant caractérisé en ce que
   (a) lesdites particules biologiques marquées passent en au moins deux zones spatialement séparées sensiblement une par une, et
   (b) ladite détection comprend la séparation de l'émission de l'agent de marquage de surface en longueurs d'onde qui ne peuvent être distinguées que par un premier moyen optique (58, 64) et un second moyen optique (59, 61, 65, 68).

2. Procédé de la revendication 1 dans lequel lesdits antigènes de surface des particules biologiques sont des antigènes de surface de cellules, et dans lequel

   - on marque de façon sélective les antigènes de surface de cellules dans un échantillon avec au moins trois taches immunofluorescentes différentes, chaque tache ayant un fluorochrome différent avec des spectres d'émission prédéterminés, essentiellement séparés;
   - on fait passer lesdits antigènes de surface de cellules marqués, essentiellement un par un, à travers une première zone de stimulation optique concentrée pour fournir une énergie d'excitation afin d'exciter une ou plusieurs desdites taches différentes;
   - on fait passer successivement lesdits antigènes de surface de cellules marqués, essentiellement un par un, à travers une seconde zone de stimulation optique concentrée pour fournir une énergie d'excitation afin d'exciter, dans ladite seconde zone, une ou plusieurs desdites taches différentes de manière qu'au moins trois taches différentes soient excitées par passage à travers lesdites deux zones de stimulation optique, la stimulation optique concentrée dans chacune desdites zones étant provoquée par la fourniture de lumière à une longueur d'onde différente à chacune desdites zones, la lumière qui est fournie à l'une desdites zones de stimulation lumineuse étant à une longueur d'onde qui provoque l'excitation de taches multiples sur les antigènes de surface cellulaires qui passent à travers ladite zone de lumière, cette longueur d'onde étant cependant suffisamment spectralement séparée de la longueur d'onde fournie à ladite autre zone de stimulation lumineuse pour être sensiblement à l'extérieur de l'intervalle spectral d'excitation et d'émission des taches excitées dans ladite autre zone de stimulation;

- on détecte la fluorescence émise par chaque tache différemment excitée par ledit premier moyen optique (58, 64) et par ledit second moyen optique (59, 61, 65, 68), séparant les émissions des taches, et ladite détection étant effectuée simultanément lorsque plusieurs signaux de fluorescence sont émis par différentes taches excitées dans l'une quelconque desdites zones de stimulation; et

- on distingue lesdits antigènes de surface cellulaires relativement à leurs caracatéristiques de fluorescence détectées.

3. Procédé des revendications 1 ou 2 dans lequel le premier moyen optique (58, 64) est constitué de miroirs dichroïques et le second moyen optique (59, 61, 65, 68) est constitué de filtres.

4. Procédé de la revendication 2 dans lequel les trois différents fluorochromes des taches sont la fluorescéine, la phycoérythrine et le rouge du Texas ou la fluorescéine, la phycoérythrine et l'allo-phycocyanine.

5. Procédé de la revendication 2 dans lequel les trois différents fluorochromes des taches sont la fluorescéine, la phycoérythrine et la R-phycocyanine.

6. Procédé des revendications 1 et 2 dans lequel lesdites cellules sont marquées avec quatre taches différentes et il existe deux zones de stimulation optique concentrée.

7. Procédé de la revendication 6 dans lequel les quatre différents fluorochromes des taches sont la fluorescéine, la phycoérythrine, le rouge du Texas et l'allo-phycocyanine.

8. Procédé de la revendication 6 dans lequel les quatre différents fluorochromes des taches sont la fluorescéine, la phycoérythrine, la R-phycocyanine et l'allo-phycocyanine.

9. Procédé des revendications 1 à 8 dans lequel la stimulation optique concentrée dans chacune desdites zones est provoquée par la fourniture d'une lumière cohérente à une longueur d'onde donnée à chacune desdites zones.

10. Procédé de la revendication 9 dans lequel la lumière cohérente fournie à une zone de stimulation est à une longueur d'onde suffisamment séparée de la longueur d'onde d'une région de stimulation spectrale adjacente pour être essentiellement à l'extérieur de l'intervalle spectral d'excitation et d'émission des taches dans une région adjacente.

11. Appareil de détection en corrélation simultanée d'antigènes de surface de cellules différents qui ont été sélectivement marqués avec des taches immunofluorescentes différentes ayant chacune des spectres d'émission prédéterminés essentiellement séparés comprenant:

- un moyen (40) pour déplacer lesdits antigènes de surface de cellules marqués dans une voie d'écoulement (38);

- un premier moyen (12) incluant une première source de lumière (12) pour exciter une ou plusieurs taches immunofluorescentes dans une première zone de stimulation optique le long de ladite voie d'écoulement (38);

- un second moyen (14) incluant une seconde source de lumière (14) pour exciter plusieurs desdites taches immunofluorescentes différentes dans une seconde zone de stimulation optique le long de ladite voie d'écoulement (38), lesdites première et seconde sources de lumière choisies pour fournir de la lumière à différentes longueurs d'onde relativement l'une à l'autre, chacune étant choisie pour provoquer l'excitation de taches multiples sur les antigènes de surface de cellules passant à travers les zones de stimulation respectives, mais qui sont suffisamment spectralement séparées l'une de l'autre pour être sensiblement à l'extérieur de l'intervalle spectral d'excitation et d'émission des taches excitées dans chacune des zones respectives de stimulation; et

- un moyen pour distinguer les antigènes de surface cellulaires relativement à leurs caractéristiques de fluorescence détectées,

ledit appareil étant caractérisé en ce que ledit organe (40) est disposé pour déplacer lesdits antigènes de surface de cellules marqués dans une voie d'écoulement (38) sensiblement un par un et ledit appareil comprenant en outre des moyens (60, 62, 66, 69) pour détecter la fluorescence émise par chaque tache immunofluorescente différemment excitée associée aux antigènes de surface des cellules y compris des moyens pour séparer plusieurs si-

gnaux de fluorescence spectralement diffé-
rents de longueur d'onde pouvant être distinguée et émis par des taches différentes excitées par l'un quelconque desdits organes d'excitation, ledit organe de séparation ne comprenant qu'un premier moyen optique (58, 64) et
un second moyen optique (59, 61, 65, 68).

12. Appareil de la revendication 11 dans lequel le
premier moyen optique (58, 64) est constitué
de miroirs dichroïques et le second moyen
optique (59, 61, 65, 68) est constitué de filtres.

13. Appareil de la revendication 11 dans lequel le
premier moyen (12) et le second moyen (14)
d'excitation comprennent chacun une source
pour fournir de la lumière cohérente auxdites
zones respectives de stimulation.

14. Appareil de la revendication 13 dans lequel les
sources (12, 14) de lumière cohérente sont
choisies pour fournir de la lumière à des longueurs d'onde suffisamment séparées les unes
des autres pour être sensiblement à l'extérieur
de l'intervalle spectral d'excitation et d'émission des taches dans chacune des zones de
stimulation respectives.

15. Appareil de la revendication 13 dans lequel la
première source (12) de lumière cohérente est
un laser à argon ionique et la seconde souce
(14) de lumière cohérente est un laser à colorant rhodamine 6-G ou un laser à l'hélium-
néon.

16. Appareil des revendications 13 et 14 dans lequel le moyen de détection comprend jusqu'à
deux dispositifs photodétecteurs associés à
chacun des premier et second organe d'excitation, chacun de ces dispositifs photodétecteurs
étant capable de détecter la lumière dans une
région de couleur spécifiquament définie.

17. Appareil de la revendication 13 dans lequel le
moyen de distinction comprend un organe
d'affichage pour indiquer les sous-populations
de cellules détectées.

18. Appareil de la revendication 13 qui comprend
en outre un moyen pour déterminer le nombre
approximatif de cellules détectées dans chaque sous-population.

**Patentansprüche**

1. Verfahren für die gleichzeitige korrelierte Bestimmung verschiedener Antigene auf der
Oberfläche biologischer Teilchen in einer Probe, umfassend

- die selektive Markierung von Antigenen
auf der Oberfläche biologischer Teilchen
in einer Probe mit einer Vielzahl ver-
schiedener Oberflächen-Markierungsmit-
tel, die auf Lichtanregung reagieren, wo-
bei jedes Markierungsmittel spektral un-
terscheidbare, quantifizierbare Markie-
rungseigenschaften aufweist, die durch
ihre Lichtabgabe meßbar sind;
- das sukzessive Hindurchschicken dieser
markierten Antigene auf der Oberfläche
biologischer Teilchen durch eine Vielzahl
räumlich getrennter Bereiche, wobei je-
der Bereich einen Lichtstrahl verschiede-
ner Wellenlänge einschließt, und das
Licht in einem Bereich eine Wellenlänge
hat, die die Anregung einer Vielzahl von
Oberflächen-Markierungsmitteln auf Antigenen auf der Oberfläche biologischer
Teilchen hervorruft, die diesen einen Bereich passieren, die aber im Spektrum
genügend weit von der Wellenlänge des
Lichts in einem benachbarten Bereich
entfernt liegt, so daß sie im wesentlichen
außerhalb des Spektralbereichs der Anregung und der Lichtabgabe der
Oberflächen-Markierungsmittel liegt, die
in einem benachbarten Bereich angeregt
werden;
- die Bestimmung der Eigenschaften einer
Anzahl von Oberflächen-Markierungsmitteln, die größer ist als die Anzahl der
Bereiche, die die Teilchen passieren, wo-
bei die Bestimmung gleichzeitig bezüg-
lich einer Vielzahl von Eigenschaften vorgenommen wird, die mit den verschiede-
nen Oberflächen-Markierungsmitteln in ir-
gendeinem dieser Bereiche verknüpft
sind; und
- die Unterscheidung verschiedener Oberflächenantigene unter den Antigenen auf
der Oberfläche biologischer Teilchen bezüglich der erfaßten, verschiedenen und
separat quantifizierbaren Eigenschaften
dieser Oberflächen-Markierungsmittel;
wobei das Verfahren dadurch gekennzeichnet
ist, daß
(a) die markierten biologischen Teilchen we-
nigstens zwei räumlich voneinander ge-
trennte Bereiche im wesentlichen einzeln
nacheinander passieren; und
(b) die Bestimmung die Trennung der Emission des Oberflächen-Markierungsmittels in
unterscheidbare Wellenlängen erst durch
die erste optische Vorrichtung (58,64) und
die zweite optische Vorrichtung
(59,61,65,68) einschließt.

2. Verfahren nach Anspruch 1, wobei die Antigene auf der Oberfläche biologischer Teilchen Antigene auf Zelloberflächen sind und das Verfahren umfaßt:

- die selektive Markierung von Antigenen auf Zelloberflächen in einer Probe mit wenigstens drei immunfluoreszierenden Flecken, wobei jeder Fleck einen verschiedenen Fluorochrom mit mit vorbestimmtem, im wesentlichen getrenntem Emissionsspektrum aufweist;

- das Hindurchschicken der markierten Antigene auf Zelloberflächen - im wesentlichen einzeln nacheinander - durch einen ersten Bereich gebündelter optischer Anregung, um Anregungsenergie zu liefern, so daß einer oder mehrere dieser verschiedenen Flecke angeregt wird;

- das sequenzweise Hindurchschicken der markierten Antigene auf Zelloberflächen - im wesentlichen nacheinander - durch einen zweiten Bereich gebündelter optischer Anregung, um Anregungsenergie zu liefern, um in diesem zweiten Bereich einen oder mehrere dieser verschiedenen Flecke anzuregen, so daß wenigstens drei verschiedene Flecke beim Durchgang durch die beiden Bereiche optischer Anregung angeregt werden, wobei die gebündelte optische Anregung in jedem der Bereiche durch Licht verschiedener Wellenlänge hervorgerufen wird, wobei das Licht, mit dem einer der Bereiche der Lichtanregung versehen wird, eine Wellenlänge aufweist, die die Anregung einer Vielzahl von Flecken auf den Zelloberflächen-Antigenen hervorruft, die den einen Lichtbereich passieren, die aber im Spektrum genügend weit von der Wellenlänge des Lichts entfernt liegt, mit dem der andere Bereich der Lichtanregung versehen wird, so daß sie im wesentlichen außerhalb des Spektralbereichs der Anregung und Emission der Flecke liegt, die in dem benachbarten Anregungsbereich angeregt werden;

- die Bestimmung der durch jeden unterschiedlich angeregten Fleck emittierten Fluoreszenz durch die erste optische Vorrichtung (58,64) und die zweite optische Vorrichtung (59,61,65,68), welche die Fleck-Emissionen trennen, wobei die Bestimmung gleichzeitig erfolgt, wenn eine Vielzahl von Fluoreszenz-Signalen von verschiedenen Flecken emittiert wird, die in irgendeinem der Anregungsbereiche angeregt werden; und

- die Unterscheidung der Zelloberflächen-Antigene bezüglich ihrer erfaßten Fluoreszenz-Eigenschaften.

3. Verfahren nach Anspruch 1 oder 2, wobei die ersten optischen Vorrichtungen (58,64) dichroitische Spiegel sind, und die zweiten optischen Vorrichtungen (59,61,65,68) Filter sind.

4. Verfahren nach Anspruch 2, wobei die drei verschiedenen Fluorochrome der Flecke Fluorescein, Phycoerythrin und Texas-Rot oder Fluorescein, Phycoerythrin und allo-Phycocyanin sind.

5. Verfahren nach Anspruch 2, wobei die drei verschiedenen Fluorochrome der Flecke Fluorescein, Phycoerythrin und R-Phycocyanin sind.

6. Verfahren nach Anspruch 1 und 2, wobei die Zellen mit vier verschiedenen Flecken markiert werden und zwei Bereiche gebündelter optischer Anregung vorhanden sind.

7. Verfahren nach Anspruch 6, wobei die vier Verschiedenen Fluorochrome der Flecke Fluorescein, Phycoerythrin, Texas-Rot und allo-Phycocyanin sind.

8. Verfahren nach Anspruch 6, wobei die vier verschiedenen Fluorochrome der Flecke Fluorescein, Phycoerythrin, R-Phycocyanin und allo-Phycocyanin sind.

9. Verfahren nach Ansprüchen 1 bis 8, wobei die gebündelte optische Anregung in jedem der Bereiche hervorgerufen wird, indem jeder der Bereiche mit kohärentem Licht einer bestimmten Wellenlänge versehen wird.

10. Verfahren nach Anspruch 9, wobei das kohärente Licht, mit dem ein Anregungsbereich versehen wird, eine Wellenlänge hat, die genügend weit von der Wellenlänge eines benachbarten Spektralbereichs der Anregung entfernt liegt, so daß sie im wesentlichen außerhalb des Spektralbereichs der Anregung und Emission der Flecke in einem benachbarten Bereich liegt.

11. Gerät für die gleichzeitige korrelierte Bestimmung verschiedener Antigene auf Zelloberflächen, die selektiv mit verschiedenen immunfluoreszierenden Flecken markiert wurden, welche jeweils vorbestimmte, im wesentlichen getrennte Emissionsspektren aufweisen, umfassend:

- Vorrichtungen (40) zum Bewegen der markierten Zelloberflächen-Antigene in einen Flußweg (38);

- erste Vorrichtungen (12), die eine erste Lichtquelle (12) zur Anregung einer oder mehrerer immunfluoreszierender Flecke in einem ersten Bereich optischer Anregung längs des Flußwegs (38) einschließen;

- zweite Vorrichtungen (14), die eine zweite Lichtquelle (14) zur Anregung einer Vielzahl der verschiedenen immunfluoreszierenden Flecke in einem zweiten Bereich optischer Anregung längs des Flußwegs (38) einschließen, wobei die erste und die zweite Lichtquelle so ausgewählt ist, daß jeweils Licht verschiedener Wellenlängen geliefert wird, und die Lichtquellen jeweils so ausgewählt werden, daß Anregung einer Vielzahl von Flecken auf Antigenen auf Zelloberflächen hervorgerufen wird, welche die jeweiligen Anregungsbereiche passieren, die aber genügend weit spektral voneinander getrennt sind, so daß sie im wesentlichen außerhalb des Spektralbereichs der Anregung und Emission der angeregten Flecke in jedem der jeweiligen Anregungsbereiche liegen; und

- Vorrichtungen zur Unterscheidung von Antigenen auf Zelloberflächen bezüglich ihrer erfaßten Fluoreszenzeigenschaften; wobei das Gerät dadurch gekennzeichnet ist, daß die Vorrichtungen (40) so angeordnet sind, daß die markierten Antigene auf Zelloberflächen im wesentlichen einzeln nacheinander in einen Flußweg (38) eingebracht werden, und das Gerät die weiteren Vorrichtungen (60,62,66,69) zur Bestimmung der Fluorezenz umfaßt, die von jedem unterschiedlich angeregten Fleck emittiert wird, der mit den Antigenen auf Zelloberflächen verknüpft ist, wozu auch Vorrichtungen zur Trennung einer Vielzahl spektral verschiedener Fluoreszenz-Signale unterscheidbarer Wellenlängen gehören, die von verschiedenen Flecken emittiert werden, die durch irgendeine der Anregungsvorrichtungen angeregt wurden, wobei diese Trennungsvorrichtungen nur erste optische Vorrichtungen (58,64) und zweite optische Vorrichtungen (59,61,65,68) umfassen.

12. Gerät nach Anspruch 11, wobei die ersten optischen Vorrichtungen (58,64) dichroitische Spiegel sind, und die zweiten optischen Vorrichtungen (59,61,65,68) Filter sind.

13. Gerät nach Anspruch 11, wobei die ersten Vorrichtungen (12) und die zweiten Vorrichtungen (14) für die Anregung jeweils eine Quelle einschließen, die für die jeweiligen Anregungsbereiche kohärentes Licht liefert.

14. Gerät nach Anspruch 13, wobei die Quellen (12,14) kohärenten Lichts so ausgewählt sind, daß sie Licht mit Wellenlängen liefern, die genügend weit spektral voneinander getrennt sind, so daß sie im wesentlichen außerhalb des Spektralbereichs der Anregung und Emission der Flecke in jedem der jeweiligen Anregungsbereiche liegen.

15. Gerät nach Anspruoh 13, wobei die erste Quelle (12) kohärenten Lichts ein Argon-Ionenlaser ist, und die zweite Quelle kohärenten Lichts ein Rhodamin-6-G-Farblaser oder ein Helium-Neon-Laser ist.

16. Gerät nach Anspruch 13 und 14, wobei die Bestimmungsvorrichtungen bis zu zwei Photodetektor-Einrichtungen einschließen, die mit jeder der ersten und zweiten Anregungsvorrichtungen verbunden sind, wobei jede dieser Photodetektor-Einrichtungen befähigt ist, Licht einer spezifisch definierten Farbregion zu bestimmen.

17. Gerät nach Anspruch 13, wobei die Unterscheidungsvorrichtungen Anzeigevorrichtungen zum Anzeigen der erfaßten Zell-Subpopulationen einschließen.

18. Gerät nach Anspruch 13, das weitere Vorrichtungen zur Bestimmung der ungefähren Anzahl erfaßter Zellen in jeder Subpopulation einschließt.

FIG.1

FIG.2

# FIG.3

ALL CELLS

TEXAS
RED

FITC

# FIG.4

LEU 2 (+)

TEXAS
RED

FITC

FIG. 5